# EUROPEAN PATENT SPECIFICATION

(11) **EP 4 403 641 B1**
(45) Date of publication and mention of the grant of the patent: **10.12.2025**
(21) Application number: 22869281.0
(22) Date of filing: 14.09.2022
(51) Int. Cl.: C12Q 1/34, C12Q 1/40, C12Q 1/48, C12Q 1/527, G01N 27/447, G01N 21/64

(54) **PROSTATE CANCER DETECTION REAGENT AND USE THEREOF IN PROSTATE CANCER DETECTION**
REAGENS ZUM NACHWEIS VON PROSTATAKREBS UND VERWENDUNG DAVON BEIM NACHWEIS VON PROSTATAKREBS
RÉACTIF DE DÉTECTION DU CANCER DE LA PROSTATE ET SON UTILISATION DANS LA DÉTECTION DU CANCER DE LA PROSTATE

(30) Priority: 15.09.2021 CN 202111079840
(43) Date of publication of application: 24.07.2024
(73) Proprietor: Jiangsu Xiansida Biotechnology Co., Ltd., Taizhou, Jiangsu 225312 (CN); Xiansida Nanjing Biotechnology Co., Ltd., Nanjing, Jiangsu 211800 (CN)
(72) Inventor: CHEN, Cuiying, Nanjing, Jiangsu 211800 (CN); WANG, Lei, Nanjing, Jiangsu 211800 (CN); TAN, Zongnan, Nanjing, Jiangsu 211800 (CN)
(74) Representative: Proi World Intellectual Property GmbH
(86) International application number: PCT/CN2022/118801
(87) International publication number: WO 2023/040912

(56) References cited:
- CN-A- 102 565 318
- CN-A- 104 807 998
- CN-A- 106 950 379
- CN-A- 106 950 380
- CN-A- 107 505 295
- CN-A- 107 894 483
- CN-A- 109 100 410
- CN-A- 109 100 507
- CN-A- 114 032 282
- R. SALDOVA ET AL: "Core fucosylation and 2-3 sialylation in serum N-glycome is significantly increased in prostate cancer comparing to benign prostate hyperplasia", GLYCOBIOLOGY, vol. 21, no. 2, 1 February 2011 (2011-02-01), pages 195 - 205, XP055091799, ISSN: 0959-6658, DOI: 10.1093/glycob/cwq147
- K. FUKUSHIMA, T. SATOH, S. BABA, K. YAMASHITA: " 1,2-Fucosylated and -N-acetylgalactosaminylated prostate-specific antigen as an efficient marker of prostatic cancer", GLYCOBIOLOGY, OXFORD UNIVERSITY PRESS, vol. 20, no. 4, 1 April 2010 (2010-04-01), pages 452 - 460, XP055035779, ISSN: 09596658, DOI: 10.1093/glycob/cwp197

## Description

### Technical field

The invention belongs to the field of biomedicine technology and involves a method for detecting prostate cancer, in particular a detection method for prostate cancer based on the spectrum of serum glycoprotein oligosaccharide detection (G-Test).

### Background technique

Prostate cancer is an epithelial malignant tumor occurring in the prostate, and it is the most common malignant tumor of the male genitourinary system. Prostate cancer progresses particularly slowly, making it difficult to detect in its early stages. The main clinical manifestations of patients include urinary difficulties, lumbago, urgent urination, frequent urination, urination pain, and other urinary tract symptoms. Treatment primarily involves radical resection, surgery, or medical castration. Early-stage prostate cancer can be cured, while conservative treatment is preferred for advanced cases.

The cause of prostate cancer is related to genetics, environment, food, and age, there is a family history of prostate cancer, the incidence is relatively high, and the age of onset will be younger. Prostate cancer tends to occur in older men over the age of 65, people with unhealthy lifestyles, and people who have had prostate cancer in their immediate family, and factors such as diet and obesity are easily induced.

In terms of epidemiology, the incidence of prostate cancer 10 years ago in our country was about 0.005%-0.006%, and the growth rate in the last 10 years in our country is relatively fast. In the past, prostate cancer was a highly common disease in Western countries, especially in the United States, where the incidence of men ranked first and the mortality rate ranked second. The incidence of prostate cancer continues to rise globally, with nearly 1.3 million new cases and 359,000 deaths worldwide in 2018. The incidence of malignant tumors in men is much higher than the mortality rate, and the risk of prostate cancer increases with age.

At present, the most important auxiliary diagnosis for prostate cancer patients is prostate-specific antigen (PSA) detection; However, this method is not sensitive to early prostate cancer, and when it is found, it is basically in the middle and late stages, and the prognosis is poor. The other way is biopsy for pathological anatomy; Of these two ways, the former is not sensitive, and the latter is more traumatic.

Glycosylation is the most common post-translational modification of protein. Glycosylation is a process in which glycans are transferred to proteins and special amino acid residues on proteins to form glycoside bonds under the action of glycosyltransferase. Most glycoproteins are secreted proteins and are widely present in cell membranes, interstitial cells, plasma, and mucus. Some enzymes and hormones are glycoproteins. Glycoproteins have many biological functions. Some glycoproteins such as procollagen are structural proteins. Many enzymes and hormones (such as luteinizing hormone, thyrotropin, etc.) have a glycoprotein structure, and many glycoproteins in the blood are responsible for the transport of inorganic ions (Fe2+, Ca2+, Cu2+, etc.) and hormones and other bioactive substances, blood coagulation (fibrinogen is a glycoprotein) and antibody activity. Lectins have the ability to agglutinate cells, and the oligosaccharide can also play the role of stabilizing the peptide chain. Another important function of glycoproteins is to participate in various recognition phenomena on the cell surface directly or indirectly. Because of the importance of the glycan in the glycoprotein to maintain the biological function of the body, the change of the glycan can help to elucidate the molecular mechanism of abnormal biological behavior such as inflammation, invasion, and metastasis of tumor cells to surrounding tissues. At present, changes in the N-glycan have been found in a variety of tumors. For example, "Core fucosylation and 2-3 sialylation in serum Nglycome is significantly increased in prostate cancer comparing to benign prostate hyperplasia" is disclosed by R. Saloova Et Al.

Oligosaccharide is an important biological information molecule, which plays a unique role in many physiological and pathological processes. The structure of oligosaccharides is very complex and exhibits microscopic heterogeneity, and its analysis and structural analysis have been the bottleneck of glycobiology research. At present, the analysis methods of oligosaccharide structure have developed rapidly, mainly including (1) high-performance liquid chromatography (HPLC): High resolution, fast detection speed, high repeatability, HPLC columns can be used repeatedly, but the column efficiency will be reduced with the increase of the number of uses, and the mobile phase is toxic, equipment operation requires highly trained professionals and the equipment is relatively expensive, and the solvent needs to be strictly purified; (2) Mass spectrometry (MS): MS has the advantages of high sensitivity, can obtain a variety of structural information and suitable for analysis of mixtures, and is an ideal means of qualitative and quantitative analysis of oligosaccharide. However, MS is precise, the equipment operation is complex, and the mass spectrometer is expensive, which is not suitable for clinical popularization and use; (3) Capillary electrophoresis (CE): CE has low cost, high column efficiency, high sensitivity, fast speed, low injection volume and simple operation, but the repeatability is not high, and the stability is not as good as HPLC.

G-Test (Glycan-Test) is a DNA analyzer-based capillary microelectrophoresis technology (DSA-FACE). The N-glycan of glycoproteins in prostate fluid samples is fluorescence-labeled and then separated by capillary microelectrophoresis. The content of N-glycan obtained by measuring the fluorescence signal is the fingerprint (referred to as the G-Test). The detection technology has the advantages of high sensitivity, simple operation, small amount (2µL serum), high repeatability, good stability, high throughput (96-well plate), and other oligosaccharide analysis technology can not be compared, which is suitable for general laboratory departments, and is expected to be used in clinical promotion.

### Content of invention

The invention is set out in the appended set of claims. The purpose of the invention is to provide a prostate cancer monitoring reagent, through which the serum glycan profile is measured, the peak value is quantified, and statistical analysis is carried out, thus providing a method for establishing the serum glycan profile model of prostate cancer.

The technical scheme of the invention is as follows:
A prostate cancer monitoring reagent comprises the following reagents:
Reagent A: The solution of ammonium bicarbonate with a concentration of 10 mM is prepared by adding SDS with a mass concentration of 0.5~5%;
Reagent B: It is prepared by mixing 0.01-10 U/10 µL glycoamidase and 0.01-10 U/10 µL Sialidase, and the pH value of the mixed solution is 4~9;
Reagent C: It is prepared from 8-Aminopyrene-1,3,6-trisulfonic acid trisodium salt dissolved in DMSO, the concentration is 0.01 mM~1 M;
Reagent D: Stopping solution.

The volume ratio of reagent A, reagent B, and reagent C is 2:2:1.

The method for preparing prostate cancer detection reagents includes the following steps:

### Step 1 Preparation of oligosaccharide

4µL of reagent A was added to the inactivated 2µL serum sample, denatured, cooled to room temperature, added 4µL of reagent B, and incubated for 1~6 h;

### Step 2 Labeling of oligosaccharide

2 µL of reagent C was added to the liquid obtained in step 1 for fluorescence labeling, and then 150 µL of reagent D was added to terminate the labeling reaction;

### Step 3 Oligosaccharide separation analysis

Take 10 µL of the liquid treated in step 2, use the analyzer to separate the oligosaccharide, and get the spectrum.

Preferably, the denaturation temperature in preparation of oligosaccharide in step 1 is not less than 75 °C heating, and the incubation temperature is not less than 25 °C.

Preferably, the temperature of the fluorescent label in step 2 is 50~90 °C.

### Illustrated description

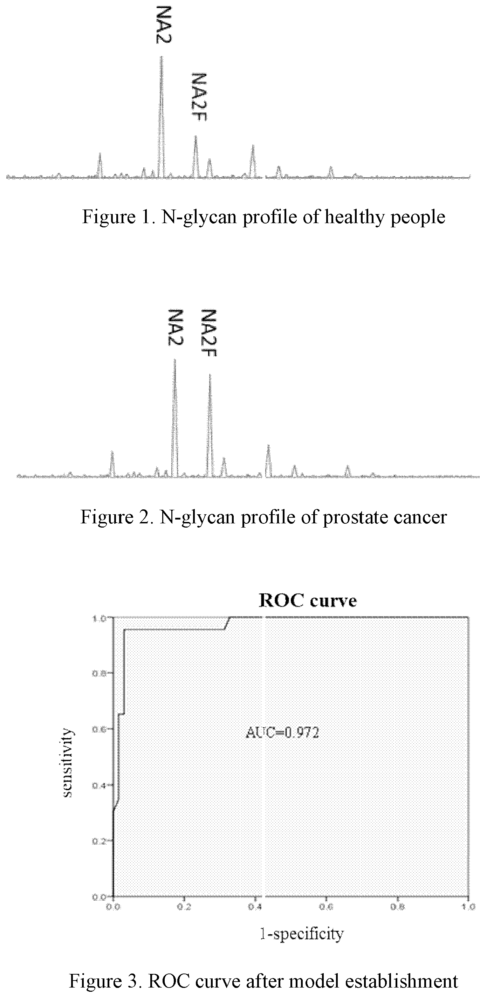

Figure 1 shows the N-glycan profile in the healthy people group. Figure 2 shows the N-glycan of serum glycoprotein in prostate cancer. The abbreviations of oligosaccharides in the graph are NA2F, Bigalacto core-α-1, 6-fucosylated biantennary; NA2, Biantennary.
Figure 3 shows the ROC curve after model establishment. The ROC curve used to identify prostate cancer was determined by the function NA2/NA2F. A total of 90 samples were detected, including 23 serum samples of prostate cancer and 67 samples of non-prostate cancer (prostatitis and prostatic hyperplasia). The AUC under the curve was 0.972.

### Concrete implementation mode

The present invention is further described below in combination with embodiments and drawings. Experimental methods not specified in the following embodiments are usually tested under conventional conditions or conditions suggested by the manufacturer, with reagents for laboratory use.

The technical scheme of the invention is as follows:
A prostate cancer monitoring reagent consists of the following reagents:
Reagent A: The solution of ammonium bicarbonate with a concentration of 10 mM is prepared by adding SDS with a mass concentration of 0.5~5%;
Reagent B: It is prepared by mixing 0.01-10 U/10 µL glycoamidase and 0.01-10 U/10 µL Sialidase, and the pH value of the mixed solution is 4~9;
Reagent C: It is prepared from 8-Aminopyrene-1,3,6-trisulfonic acid trisodium salt dissolved in DMSO, the concentration is 0.01 mM~1 M;
Reagent D: Stopping solution.

Preferably, the volume ratio of reagent A, reagent B, and reagent C is 2:2:1.

A method for preparing a prostate cancer detection reagent comprises the following steps:

### Step 1 Preparation of oligosaccharide

4µL of reagent A was added to the inactivated 2µL serum sample, denatured, cooled to room temperature, added 4µL of reagent B, and incubated for 1~6 h;

### Step 2 Labeling of oligosaccharide

2 µL of reagent C was added to the liquid obtained in step 1 for fluorescence labeling, and then 150 µL of reagent D was added to terminate the labeling reaction;

### Step 3 Oligosaccharide separation analysis

Take 10 µL of the liquid treated in step 2, use the analyzer to separate the oligosaccharide, and get the spectrum.

Preferably, the denaturation temperature in the preparation of step 1 oligosaccharides is not less than 75 ° C for heating, and the incubation temperature is not less than 25 °C.

Preferably, the fluorescently labeled temperature in step 2 is 50 to 90 °C.Application of a composition in the preparation of prostate cancer monitoring reagents, the composition detects prostate cancer by the ratio of NA2/NA2F.

Glycosylation refers to the formation of N-glycan through complex interactions between hundreds of enzymes, transcription factors, ion channels, and proteins, and is involved in a variety of molecular processes, such as protein folding, cell adhesion, molecular transfer, signal transduction, and regulation of receptor activity. Changes in N-glycan on glycoproteins are associated with disease.

A large number of previous studies have found that the relative contents of N-oligosaccharide NA2(bigalacto biantennary glycan) and NA2F(bigalacto core-alpha-1,6-fucosylated bisecting biantennary glycan) in serum of prostate cancer patients have significantly changed compared with that in normal serum, so they can be used as markers for detecting prostate cancer.

Given the existing prostate cancer detection, the specificity and accuracy of blood detection and imaging detection are not high, the pathological examination is traumatic, prone to sampling errors, and patient dependence is poor, the invention provides a prostate cancer N-glycan detection method.

### Materials and methods:

1, Test samples: serum from prostate cancer patients and healthy people.
2, Experimental equipment: oligosaccharide analyzer, PCR, centrifuge.
3, Reagent preparation:
   Reagent A: The solution of ammonium bicarbonate with a concentration of 10 mM is prepared by adding SDS with a mass concentration of 0.5~5%;
   Reagent B: It is prepared by mixing 0.01-10 U/10 µL glycoamidase and 0.01-10 U/10 µL Sialidase, and the pH value of the mixed solution is 4~9;
   Reagent C: It is prepared from 8-Aminopyrene-1,3,6-trisulfonic acid trisodium salt dissolved in DMSO, the concentration is 0.01 mM~1 M;
   Reagent D: Stopping solution.
4, Oligosaccharide sequencing test steps:
   Step 1 Preparation of oligosaccharide
      4µL of reagent A was added to the inactivated 2µL serum sample, denatured, cooled to room temperature, added 4µL of reagent B, and incubated for 1~6 h;
   Step 2 Labeling of oligosaccharide
      2 µL of reagent C was added to the liquid obtained in step 1 for fluorescence labeling, and then 150 µL of reagent D was added to terminate the labeling reaction;
   Step 3 Oligosaccharide separation analysis
      Take 10 µL of the liquid treated in step 2, use the analyzer to separate the oligosaccharide, and get the spectrum.
5, Monitoring contrastive analysis

Peak quantization was performed on the obtained N-glycan, and the relative content of each peak was quantitatively calculated by comparing the peak height value of each peak with the sum of the heights of all peaks. The composition was further statistically analyzed by the function NA2/NA2F to detect prostate cancer.

Compared with the existing technology, the invention has the following beneficial effects:
(1) The method of the invention is based on the fingerprint of detecting N-glycan in serum glycoprotein, creating a detection method and detection system for prostate cancer diagnosis. This method can allow many highly suspected prostate cancer patients to receive routine, non-invasive detection, help doctors detect, and timely monitor the occurrence and progression of the disease, and has a more obvious accuracy than other current technologies, the sensitivity of prostate cancer detection reached 97.2%.
(2) Compared with the existing technology, the detection method of the invention has an accuracy of 97.2%, which is superior to the existing detection method. The reagent of the invention can enable many high-risk people to receive routine and non-invasive detection, help doctors and patients timely monitor the occurrence and progression of prostate cancer, and is expected to be widely used in clinical practice.

### Example 1 Detection of prostate cancer

Materials and methods:
1, Test samples: serum from prostate cancer patients and healthy people.
2, Experimental equipment: capillary electrophoresis analyzer, PCR, centrifuge.
3, Reagent preparation:
   Reagent A: The solution of ammonium bicarbonate with a concentration of 10 mM was prepared by adding SDS with a mass concentration of 0.5~5%;
   Reagent B: It is prepared by mixing 0.01-10 U/10 µL glycoamidase and 0.01-10 U/10 µL Sialidase, and the pH value of the mixed solution is 4~9;
   Reagent C: It is prepared from 8-Aminopyrene-1,3,6-trisulfonic acid trisodium salt dissolved in DMSO, the concentration is 0.01 mM~1 M;
      Reagent D: Stopping solution.
4, Oligosaccharide sequencing test steps:
   Step 1 Preparation of oligosaccharide
      4µL of reagent A was added to the inactivated 2µL serum sample, denatured, cooled to room temperature, added 4µL of reagent B, and incubated for 1~6 h;
   Step 2 Labeling of oligosaccharide
      2 µL of reagent C was added to the liquid obtained in step 1 for fluorescence labeling, and then 150 µL of reagent D was added to terminate the labeling reaction;
   Step 3 Oligosaccharide separation analysis
      Take 10 µL of the liquid treated in step 2, use the analyzer to separate the oligosaccharide, and get the spectrum.
5, Monitoring contrastive analysis

Serum samples collected from 90 patients with prostate cancer and healthy people were processed by the G-Test technique, including 23 with prostate cancer and 67 with non-prostate cancer (prostatitis and prostatic hyperplasia). The N-glycan profile obtained by the G-Test was analyzed statistically.

The peak height of each peak was divided by the sum of all peak heights, and the relative content of each peak was obtained by quantitative calculation, that is, the peak value of the N-glycan profile was quantified, and then the 9 oligosaccharide peaks in the N-glycan profile of the quantified prostate cancer group and the healthy people group were compared and statistically analyzed. As shown in Fig. 1 and Fig. 2, the N-glycan profile of human serum shows nearly 9 peaks of N-glycan, and the mobility of oligosaccharide chains varies according to the molecular size, that is, different peaks on the N-glycan profile represent different oligosaccharide, and the measured peak height represents the relative concentration of oligosaccharide. The N-glycan profile was further statistically analyzed by the ratio of NA2/NA2F to detect prostate cancer.

The ROC curve used to identify prostate cancer was determined by the function NA2/NA2F. The total number of samples was 90, including 23 serum samples of prostate cancer and 67 samples of non-prostate cancer (prostatitis and prostatic hyperplasia). The area under the curve AUC=0.972.

Compared with the existing technology, the detection method of the invention has an accuracy of 97.2%, which is superior to the existing detection method. The reagent of the invention can enable many high-risk people to receive routine and non-invasive detection, help doctors and patients timely monitor the occurrence and progression of prostate cancer, and is expected to be widely used in clinical practice.

The content within the above detailed description to the embodiment which does not form part of the claim(s) is for the purpose of understanding the present invention only.

The specific embodiments described above, combined with the accompanying drawings, provide further detailed explanations of the purpose, technical scheme, and beneficial effects of the invention.

## Claims

1. A prostate cancer monitoring reagent **characterized in that** it comprises the following reagents:
Reagent A: The solution of ammonium bicarbonate with a concentration of 10 mM is prepared by adding SDS with a mass concentration of 0.5~5%;
Reagent B: It is prepared by mixing 0.01-10 U/10 µL glycoamidase and 0.01-10 U/10 µL Sialidase, and the pH value of the mixed solution is 4~9;
Reagent C: It is prepared from 8-Aminopyrene-1,3,6-trisulfonic acid trisodium salt dissolved in DMSO, the concentration is 0.01 mM~1 M;
Reagent D: Stopping solution.

2. The prostate cancer detection reagent according to claim 1, **characterized in that** volume ratio of reagent A, reagent B, and reagent C is 2:2:1.

3. A method for preparing prostate cancer detection reagents according to claim 1, **characterized by** including the following steps:
Step 1 Preparation of oligosaccharide
4µL of reagent A was added to the inactivated 2µL serum sample, denatured, cooled to room temperature, added 4µL of reagent B, and incubated for 1~6 h;
Step 2 Labeling of oligosaccharide
2 µL of reagent C was added to the liquid obtained in step 1 for fluorescence labeling, and then 150 µL of reagent D was added to terminate the labeling reaction;
Step 3 Oligosaccharide separation analysis
Take 10 µL of the liquid treated in step 2, use the analyzer to separate the oligosaccharide, and get the spectrum.

4. The method for preparing prostate cancer detection reagent according to claim 3, **characterized in that** the denaturation temperature in preparation of oligosaccharide in step 1 is not less than 75 °C heating, and the incubation temperature is not less than 25 °C.

5. The method for preparing prostate cancer detection reagent according to claim 3, **characterized in that** the temperature of the fluorescent label in step 2 is 50~90 °C.

## Patentansprüche

1. Reagenz zur Überwachung von Prostatakrebs, **dadurch gekennzeichnet, dass** es die folgenden Reagenzien umfasst:
Reagenz A: Die Lösung von Ammoniumbicarbonat mit einer Konzentration von 10 mM wird durch Zugabe von SDS mit einer Massenkonzentration von 0,5 bis 5 % hergestellt.
Reagenz B: Es wird durch Mischen von 0,01-10 U/10 µl Glycoamidase und 0,01-10 U/10 µl Sialidase hergestellt, wobei der pH-Wert der gemischten Lösung 4 bis 9 beträgt.
Reagenz C: Es wird aus 8-Aminopyren-1,3,6-trisulfonsäure-Trinatrium-Salz hergestellt, das in DMSO gelöst ist, die Konzentration beträgt 0,01 mM bis 1 M.
Reagenz D: Stopplösung.

2. Reagenz zum Nachweis von Prostatakrebs gemäß Anspruch 1, **dadurch gekennzeichnet, dass** das Volumenverhältnis von Reagenz A, Reagenz B und Reagenz C 2:2:1 beträgt.

3. Verfahren zur Herstellung von Reagenzien zum Nachweis von Prostatakrebs gemäß Anspruch 1, **gekennzeichnet durch** die folgenden Schritte:
Schritt 1 Herstellung von Oligosaccharid
4 µl Reagenz A wurden zu der inaktivierten 2-µl-Serumprobe gegeben, denaturiert, auf Raumtemperatur abgekühlt, 4 µl Reagenz B hinzugefügt und 1 bis 6 Stunden inkubiert.
Schritt 2 Markierung des Oligosaccharids
2 µl Reagenz C wurden zu der in Schritt 1 erhaltenen Flüssigkeit zur Fluoreszenzmarkierung hinzugefügt, und dann wurden 150 µl Reagenz D hinzugefügt, um die Markierungsreaktion zu beenden.
Schritt 3 Oligosaccharid-Trennungsanalyse
10 µl der in Schritt 2 behandelten Flüssigkeit entnehmen, das Oligosaccharid mit dem Analysegerät trennen und das Spektrum erhalten.

4. Verfahren zur Herstellung eines Reagenzes zum Nachweis von Prostatakrebs gemäß Anspruch 3, **dadurch gekennzeichnet, dass** die Denaturierungstemperatur bei der Herstellung des Oligosaccharids in Schritt 1 nicht weniger als 75 °C beträgt und die Inkubationstemperatur nicht weniger als 25 °C beträgt.

5. Verfahren zur Herstellung eines Prostatakrebs-Nachweisreagenzes gemäß Anspruch 3, **dadurch gekennzeichnet, dass** die Temperatur der Fluoreszenzmarkierung in Schritt 2 50 bis 90 °C beträgt.

## Revendications

1. Réactif de surveillance du cancer de la prostate **caractérisé en ce qu'**il comprend les réactifs suivants :
Réactif A : la solution de bicarbonate d'ammonium à une concentration de 10 mM est préparée en ajoutant du SDS à une concentration massique de 0,5 à 5 % ;
Réactif B : il est préparé en mélangeant 0,01 à 10 U/10 µL de glycoamidase et 0,01 à 10 U/10 µL de sialidase, et le pH de la solution mélangée est compris entre 4 et 9 ;
Réactif C : il est préparé à partir de sel trisodique d'acide 8-aminopyrene-1,3,6-trisulfonique dissous dans du DMSO, la concentration est de 0,01 mM à 1 M;
Réactif D : solution d'arrêt.

2. Réactif de détection du cancer de la prostate selon la revendication 1, **caractérisé en ce que** le rapport volumique entre le réactif A, le réactif B et le réactif C est de 2:2:1.

3. Procédé de préparation de réactifs de détection du cancer de la prostate selon la revendication 1, **caractérisé en ce qu'**il comprend les étapes suivantes :
Étape 1 Préparation de l'oligosaccharide
4 µL de réactif A ont été ajoutés à l'échantillon de sérum inactivé de 2 µL, dénaturés, refroidis à température ambiante, 4 µL de réactif B ont été ajoutés et incubés pendant 1 à 6 heures ;
Étape 2 Marquage de l'oligosaccharide
2 µL de réactif C ont été ajoutés au liquide obtenu à l'étape 1 pour le marquage par fluorescence, puis 150 µL de réactif D ont été ajoutés pour terminer la réaction de marquage ;
Étape 3 Analyse de séparation des oligosaccharides
Prélever 10 µL du liquide traité à l'étape 2, utiliser l'analyseur pour séparer l'oligosaccharide et obtenir le spectre.

4. Procédé de préparation d'un réactif de détection du cancer de la prostate selon la revendication 3, **caractérisé en ce que** la température de dénaturation lors de la préparation de l'oligosaccharide à l'étape 1 n'est pas inférieure à 75 °C et la température d'incubation n'est pas inférieure à 25 °C.

5. Procédé de préparation d'un réactif de détection du cancer de la prostate selon la revendication 3, **caractérisé en ce que** la température du marqueur fluorescent à l'étape 2 est comprise entre 50 et 90 °C.
